# EUROPEAN PATENT APPLICATION

(11) **EP 4 643 854 A1**
(43) Date of publication of application: **05.11.2025**
(21) Application number: 23912805.1
(22) Date of filing: 22.12.2023
(51) Int. Cl.: A61K 9/70, A61K 47/38, A61K 47/12, A61K 31/381, A61P 25/16

(54) **FORMULATION FOR TRANSDERMAL ABSORPTION AND METHOD FOR PREPARING SAME**

(30) Priority: 26.12.2022 KR 20220184089
(71) Applicant: Whan In Pharmaceutical Co., Ltd., Songpa-gu, Seoul 05855 (KR)
(72) Inventor: LEE, Jung Sik, Yongin-si Gyeonggi-do 16957 (KR); JANG, Bo Seul, Yongin-si Gyeonggi-do 16957 (KR); LEE, Cheol Ho, Yongin-si Gyeonggi-do 16957 (KR); KIM, Yong Han, Yongin-si Gyeonggi-do 16957 (KR); SHIN, Ho Chul, Yongin-si Gyeonggi-do 16957 (KR)
(74) Representative: Mewburn Ellis LLP
(86) International application number: PCT/KR2023/021372
(87) International publication number: WO 2024/144130

(57) **Abstract**

In a formulation for transdermal absorption and a method for preparing same according to the present invention, the formulation for transdermal absorption comprises: rotigotine or a pharmaceutically acceptable salt thereof as an active ingredient; hydroxypropyl cellulose (HPC) as a crystallization prevention agent; and a drug-containing adhesive layer comprising a silicone-based polymer adhesive.

## Description

### Technical Field

The present invention relates to a formulation for transdermal absorption containing rotigotine and a method for preparing the same.

### Background Art

Rotigotine is a non-ergoline dopamine agonist used to treat Parkinson's disease (PD) and restless legs syndrome (RLS). Such a component, the rotigotine, is commercially sold in the form of a patch formulation.

A transdermal therapeutic system containing the rotigotine in a self-adhesive matrix layer is disclosed in US 7,413,747 B, the matrix layer including an acrylate or silicone-based polymer adhesive. In commercially available patches containing rotigotine, silicone adhesives are used to improve the transdermal permeability of the rotigotine. However, the crystal precipitation of the rotigotine easily occurs when the patches containing the rotigotine are stored at room temperature because of the low solubility of the rotigotine to the silicone adhesives, resulting in challenges in securing the shelf-life required for commercial distribution. In WO 2011/076879 A, it is attempted to minimize the precipitation of rotigotine crystals by adding polyvinyl pyrrolidone polymers. However, despite these efforts, the problem of partial crystallization on some commercial products still arises. Therefore, there is a demand for methods to solve such a problem.

### Related Art References

### Patent Documents

(Patent Document 0001): US Patent No. US 7,413,747 B
(Patent Document 0002): International Publication No. WO 2011/076879 A

### Disclosure

### Technical Problem

Therefore, the present inventors have conducted studies in various ways to improve the storage stability of a transdermal patch containing rotigotine, and have found a transdermal absorption formulation in which no crystallization of the rotigotine occurs and the rotigotine is present in the formulation in a dissolved state even though a silicone adhesive is used, and a method for preparing the same, to complete the present invention.

### Solution to Problem

A transdermal absorption formulation according to the present invention contains a drug-containing adhesive layer comprises rotigotine or a pharmaceutically acceptable salt thereof as an active ingredient; hydroxypropyl cellulose (HPC) as a crystallization inhibitor; and a silicone-based polymer adhesive. The transdermal absorption formulation according to the present invention may be a transdermal patch.

In the present invention, the rotigotine refers to (-)-5,6,7,8-tetrahydro-6-[propyl-[2-(2-thienyl)ethyl]amino]-1 naphthalenol, which is a compound represented by Formula 1 below.

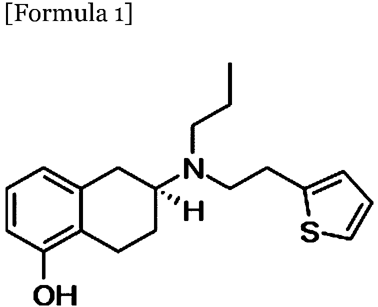

In the present invention, the pharmaceutically acceptable salt of rotigotine may be an organic acid salt, an inorganic acid salt, a metal salt, or the like, and is not particularly limited thereto.

In the present invention, the transdermal absorption formulation may contain rotigotine or the pharmaceutically acceptable salt thereof, as a free base of rotigotine, in an amount of 0.1 mg/cm² to 1.0 mg/cm².

In the present invention, the transdermal absorption formulation may contain rotigotine or the pharmaceutically acceptable salt thereof, as a free base of rotigotine, in an amount of 2% to 20% by weight with respect to 100% by weight of the total weight of the drug-containing adhesive layer in the transdermal absorption formulation.

In the present invention, the HPC is a crystallization inhibitor of rotigotine, and refers to cellulose with a degree of substitution of hydroxypropoxy groups of at least 20% by mass or more. For example, the HPC of the present invention may be cellulose with a degree of substitution of hydroxypropoxy groups of at least 50% by mass or more.

In the present invention, the HPC exhibits excellent compatibility with the silicone-based polymer adhesive, and a notable ability to inhibit rotigotine crystallization within the formulation.

In one embodiment, the HPC of the present invention may have a molecular weight of 40,000 to 1,000,000. For example, the HPC of the present invention may have a molecular weight of 40,000 to 280,000. In this case, the molecular weight may refer to weight-average molecular weight.

In one embodiment, the HPC of the present invention may have a viscosity of 2 to 4,000 mPa·s in a 2% aqueous solution at 20°C.

In the present invention, a content of the HPC may be 1% to 20% by weight with respect to 100% by weight of the total weight of the drug-containing adhesive layer in the transdermal absorption formulation. The total weight of the drug-containing adhesive layer refers to the total weight after a drug-containing adhesive composition is dried. In this case, the drug-containing adhesive composition contains rotigotine or a pharmaceutically acceptable salt thereof as an active ingredient, HPC as a crystallization inhibitor, a silicone-based polymer adhesive, and an organic solvent. In this case, the organic solvent may be an alcohol having 1 to 5 carbon atoms.

In one embodiment, the content of the HPC may be 10% to 15% by weight with respect to 100% by weight of the total weight of the drug-containing adhesive layer in the transdermal absorption formulation.

In one embodiment, a ratio of the weight of the rotigotine or the pharmaceutically acceptable salt thereof, as rotigotine, to the HPC (hereinafter, referred to as the weight ratio of rotigotine to HPC) may be 1:0.1 to 1:10. For example, the weight ratio of rotigotine to the HPC may be 1:1 to 1:5. Specifically, the weight ratio of rotigotine to the HPC may be 1:1 to 1:2.

In one embodiment, the weight ratio of rotigotine to the HPC may be 9:12 to 9:15.

In the present invention, the silicone-based adhesive can be used without particular limitations as long as it is an adhesive composition containing a silicone-based copolymer.

In one embodiment, the silicone-based adhesive in the present invention may include a standard silicone pressure-sensitive adhesive containing trimethylated silica treated with dimethyl siloxane, an amine-resistant silicone adhesive, or a mixture thereof. Examples of the standard silicone pressure-sensitive adhesive include BIO-PSA 7-4401, BIO-PSA 7-4402, BIO-PSA 7-4501, BIO-PSA 7-4502, BIO-PSA 7-4601, BIO-PSA 7-4602, and the like, examples of the amine-resistant silicone adhesive include BIO-PSA 7-4101, BIO-PSA 7-4102, BIO-PSA 7-4201, BIO-PSA 7-4202, BIO-PSA 7-4301, BIO-PSA 7-4302, and the like, and the listed components may be used alone or in combinations of two or more. The silicone-based adhesive in the present invention may be a product manufactured by Dow Corning Corporation.

In the present invention, the crystallization of rotigotine can be inhibited even though rotigotine and HPC described above are used together with the silicone-based adhesive.

In one embodiment, the transdermal absorption formulation according to the present invention may further contain a fatty acid.

In the present invention, the fatty acid can act as a solubilizer for rotigotine. In the present invention, the carboxyl group of the fatty acid serves to increase the solubility of rotigotine in formulation through ion-pairing with basic rotigotine molecules. The fatty acid according to the present invention may have a main chain with 4 to 28 carbon atoms. In one embodiment, the fatty acid according to the present invention may have a main chain with 6 to 22 carbon atoms, and specifically a main chain with 12 to 18 carbon atoms. In one embodiment, the fatty acid in the present invention may be oleic acid.

In the present invention, a content of the fatty acid may be 0.01% to 5% by weight with respect to 100% by weight of the total weight of the drug-containing adhesive layer.

A ratio of the weight of rotigotine or the pharmaceutically acceptable salt thereof , asrotigotine, to the fatty acid (hereinafter, referred to as the weight ratio of rotigotine to fatty acid) may be 1:0.001 to 1:1. For example, the weight ratio of rotigotine to fatty acid may be 1:0.001 to 1:0.7. Within the above-described weight ratio range, rotigotine component exhibits excellent transdermal permeability. The fatty acid, acting as a solubilizer, optimally enhances the solubility of an active ingredient in the adhesive layer, thereby enabling excellent skin delivery of rotigotine within the patch formulation through passive diffusion. In addition, in the transdermal patch, the adhesive layer and the backing layer can maintain a stable bond without physical separation.

In one embodiment, the weight ratio of rotigotine to the fatty acid may be 9:0.01 to 9:5. For example, the weight ratio of rotigotine and the fatty acid may be 9:0.01 to 9:4 or 9:0.01 to 9:3. Within the above-described weight ratio range, the crystallization of rotigotine may not occur. In addition, within the above-described weight ratio range, crystallization is avoided in the transdermal patch, and the adhesive layer and backing layer can maintain a stable bond without physical separation.

In one embodiment, in a case where the transdermal absorption formulation further contains the fatty acid, the content of the hydroxypropyl cellulose may be 10% to 15% by weight with respect to the total weight of the drug-containing adhesive layer, and the content of the fatty acid may be 0.01% to 5% by weight, 0.01% to 4% by weight, or 0.01% to 3% by weight.

The transdermal absorption formulation according to the present invention may further contain an antioxidant for the chemical stability of the active ingredient.

Examples of the antioxidant include tocopherol, esters thereof or derivatives thereof, ascorbic acid, ascorbyl palmitate, 2,5-dihydroxybenzoic acid, dibutylhydroxytoluene (DHT), butylated hydroxyanisole, and propyl gallate, and are not particularly limited thereto. These may be used alone or in combinations of two or more.

The transdermal absorption formulation according to the present invention may further contain polyvinyl pyrrolidone.

In one embodiment, the transdermal absorption formulation of the present invention may further include a backing layer that serves as a base of the drug-containing adhesive layer; and a release liner that protects the drug-containing adhesive layer and is removed immediately prior to use. The backing layer and the release liner are disposed on both surfaces of the drug-containing adhesive layer so that the drug-containing adhesive layer is interposed therebetween.

A method for preparing the transdermal absorption formulation according to the present invention includes: a step of coating a backing layer with a drug-containing adhesive composition that contains rotigotine or a pharmaceutically acceptable salt thereof as an active ingredient, HPC as a crystallization inhibitor, a silicone-based polymer adhesive, and an organic solvent; and
a step of drying the drug-containing adhesive composition coated onto the backing layer to form a drug-containing adhesive layer.

The organic solvent may include an alcohol having 1 to 5 carbon atoms.

The active ingredient, the crystallization inhibitor, and the polymer adhesive contained in the drug-containing adhesive composition are substantially the same as those described in the adhesive layer of the transdermal absorption formulation, respectively. Therefore, the redundant detailed descriptions thereof are not repeated.
(1) A transdermal absorption formulation according to the present invention comprising a drug-containing adhesive layer, wherein the drug-containing adhesive layer comprises rotigotine or a pharmaceutically acceptable salt thereof as an active ingredient; hydroxypropyl cellulose (HPC) as a crystallization inhibitor; and a silicone-based polymer adhesive.
(2) According to (1), a weight ratio of rotigotine or the pharmaceutically acceptable salt thereof, as rotigotine, to the HPC may be 1:0.1 to 1:10.
(3) According to (1) or (2), a weight ratio of rotigotine or the pharmaceutically acceptable salt thereof, as rotigotine, tothe HPC may be 9:12 to 9:15.
(4) According to any one of (1) to (3), the drug-containing adhesive layer may further comprise a fatty acid as a solubilizer.
(5) According to (4), a weight ratio of rotigotine or the pharmaceutically acceptable salt thereof, as rotigotine, to the fatty acid may be 9:0.01 to 9:5.
(6) According to (4) or (5), a content of the hydroxypropyl cellulose may be 10% to 15% by weight with respect to a total weight of the drug-containing adhesive layer, and a content of the fatty acid may be 0.01% to 5% by weight with respect to the total weight of the drug-containing adhesive layer.
(7) According to any one of (1) to (6), a/the content of the hydroxypropyl cellulose may be 10% to 15% by weight with respect to a total weight of the drug-containing adhesive layer.
(8) According to any one of (1) to (7), the drug-containing adhesive layer may further comprise an antioxidant.
(9) According to any one of (1) to (8), the drug-containing adhesive layer may further comprise polyvinyl pyrrolidone.
(10) According to any one of (1) to (9), the transdermal absorption formulation may be a transdermal patch.
(11) According to any one of (1) to (10), the transdermal absorption formulation may be a transdermal patch that comprise a backing layer and a release liner respectively disposed on both surfaces of the drug-containing adhesive layer so that the drug-containing adhesive layer is interposed between the backing layer and the release liner.
(12) A method for preparing the transdermal absorption formulation according to the present invention comprises: a step of coating a backing layer with a transdermal absorption composition that comprises rotigotine or a pharmaceutically acceptable salt thereof as an active ingredient, hydroxypropyl cellulose (HPC) as a crystallization inhibitor, a silicone-based polymer adhesive, and an organic solvent; and a step of drying a drug-containing adhesive composition coated onto the backing layer to form a drug-containing adhesive layer.
(13) According to (12), in the step of coating the backing layer with the transdermal absorption composition, the transdermal absorption composition may further contain a fatty acid.
(14) According to (12) or (13), the organic solvent may include an alcohol having 1 to 5 carbon atoms.

### Advantageous Effects

The transdermal formulation according to the present invention, even when used with a silicone-based polymer adhesive, can stably maintain the active ingredient rotigotine in a dissolved state without crystallization. Consequently, rotigotine can maintain a stable dissolved state without precipitating as crystals within the drug-containing adhesive layer of a transdermal patch including the silicone-based polymer adhesive, while simultaneously maximizing the transdermal delivery of rotigotine. Furthermore, the transdermal formulation according to the present invention also exhibits excellent skin adhesion. In particular, the transdermal absorption formulation according to the present invention can improve initial adhesion by using the silicone-based polymer adhesive together with the HPC. Moreover, the formulation maintains a stable bond between the adhesive layer and the backing layer without physical separation. When the transdermal formulation according to the present invention is applied to a user's skin, the cold flow phenomenon, where the silicone-based polymer adhesive remains on the skin contact surface, is also not observed.

### Description of Drawings

FIG. 1 illustrates microscopic photographs of transdermal patches according to Examples of the present invention.
FIG. 2 illustrates a microscopic photograph of a transdermal patch according to Comparison Example 1.
FIG. 3 illustrates a graph showing the transdermal absorption performance of the transdermal patches according to Examples 4, 6, and 7 of the present invention.

### Mode for Invention

Hereinbelow, an embodiment of the present invention will be described in detail. Unless otherwise specified, all terms used herein, including technical or scientific terms, have the same meaning as commonly understood by a person having ordinary skill in the art to which the present invention pertains. Terms such as those defined in the dictionary in general use shall be construed to have meanings consistent with the context of the related technology and shall not be construed in an ideal or overly formal sense unless it is clearly defined as such in the present application.

### Examples 1 to 24

Drug-containing adhesive layers with compositions according to Table 1 to Table 4 below were prepared in the following order. In Table 1 to Table 4, the content of each component refers to the content of each component based on 100% by weight of the drug-containing adhesive layer.

An HPC (Nisso HPC, purchased from NIPPON SODA CO., Ltd.) as a crystallization inhibitor was firstly added into a 50 mL light-shielding vial, an appropriate amount of ethanol was added to dissolve the HPC, an antioxidant dissolved in the ethanol was mixed with a fatty acid, the rotigotine was added therein and dissolved, and a silicone-based polymer adhesive purchased from DuPont de Nemours, Inc. was then added therein, followed by mixing the mixture using a magnetic bar for one hour to prepare a transdermal absorption composition.

Thereafter, the prepared transdermal absorption composition was left to stand for 30 minutes to remove the air bubbles, and a fluoropolymer-treated polyester (PET) film was coated with the transdermal absorption composition prepared above on a coating machine by using a knife coater. Thereafter, the coating was dried at 80°C for 6 minutes in an oven, and the backing layer film was applied onto the dried coating layer, pressed using a compression roller, and cut to an area of 10 cm² using a patch cutter to prepare a transdermal patch as a transdermal absorption formulation.

**[Table 1]**

| **Type** | **Example (% by weight)** | | | | | |
|---|---|---|---|---|---|---|
| | **Example 1** | **Example 2** | **Example 3** | **Example 4** | **Example 5** | **Example 6** |
| **Rotigotine** | **9** | **9** | **9** | **9** | **9** | **9** |
| **Hydroxypropyl cellulose** | **12** | **14** | **10** | **10** | **10** | **12** |
| **Oleic acid** | **0** | **0** | **0.25** | **0.5** | **1** | **0.1** |
| **Silicon pressure-sensitive adhesive (BIO-PSA^{™})** | **78.5** | **76.5** | **80.25** | **80** | **79.5** | **78.4** |
| **Butylated hydroxytoluene** | **0.3** | **0.3** | **0.3** | **0.3** | **0.3** | **0.3** |
| **Tocopherol** | **0.2** | **0.2** | **0.2** | **0.2** | **0.2** | **0.2** |
| **Total** | **100** | **100** | **100** | **100** | **100** | **100** |

**[Table 2]**

| **Type** | **Example (% by weight)** | | | | | |
|---|---|---|---|---|---|---|
| | **Example 7** | **Example 8** | **Example 9** | **Example 10** | **Example 11** | **Example 12** |
| **Rotigotine** | **9** | **9** | **9** | **9** | **9** | **9** |
| **Hydroxypropyl cellulose** | **14** | **12** | **12** | **12** | **12** | **12** |
| **Oleic acid** | **0.1** | **0.1** | **0.1** | **0.1** | **0.1** | **0.1** |
| **Silicon pressure-sensitive adhesive (BIO-PSA^{™})** | **76.4** | **78.9** | **78.76** | **78.62** | **78.6** | **78.75** |
| **Butylated hydroxytoluene** | **0.3** | **0** | **0** | **0** | **0.3** | **0.15** |
| **Tocopherol** | **0.2** | **0** | **0.14** | **0.28** | **0** | **0** |
| **Total** | **100** | **100** | **100** | **100** | **100** | **100** |

**[Table 3]**

| **Type** | **Example (% by weight)** | | | | | |
|---|---|---|---|---|---|---|
| | **Example 13** | **Example 14** | **Example 15** | **Example 16** | **Example 17** | **Example 18** |
| **Rotigotine** | **9** | **11.25** | **11.25** | **10** | **10** | **9.57** |
| **Hydroxypropyl cellulose** | **13** | **15** | **15** | **13.33** | **13.33** | **12** |
| **Oleic acid** | **0.1** | **0** | **0.1** | **0** | **0.1** | **0** |
| **Silicon pressure-sensitive adhesive (BIO-PSA^{™})** | **77.9** | **73.75** | **73.65** | **76.67** | **76.57** | **78.43** |
| **Total** | **100** | **100** | **100** | **100** | **100** | **100** |

**[Table 4]**

| **Type** | **Example (% by weight)** | | | | | |
|---|---|---|---|---|---|---|
| | **Example 19** | **Example 20** | **Example 21** | **Example 22** | **Example 23** | **Example 24** |
| **Rotigotine** | **9** | **11.25** | **11.25** | **9** | **9** | **9** |
| **Hydroxypropyl cellulose** | **13** | **15** | **15** | **12** | **12** | **12** |
| **Oleic acid** | **0.1** | **0** | **0.1** | **1** | **3** | **5** |
| **Silicon pressure-sensitive adhesive (BIO-PSA^{™})** | **77.9** | **73.75** | **73.65** | **78** | **76** | **74** |
| **Total** | **100** | **100** | **100** | **100** | **100** | **100** |

### Comparative Example 1

Comparative Example 1 was prepared substantially the same procedure as Examples 1 to 24, with the components and composition according to Table 5, except that HPC and fatty acid were not used.

**[Table 5]**

| **Type** | **Example (% by weight)** |
|---|---|
| | **Comparative Example 1** |
| **Rotigotine** | **9** |
| **Hydroxypropyl cellulose** | **0** |
| **Silicon pressure-sensitive adhesive (BIO-PSA^{™})** | **91** |
| **Total** | **100** |

### Experiment Example 1: Evaluation of Rotigotine Crystal Precipitation

Crystal precipitation and cold flow (the phenomenon in which adhesive oozes to the edge of the cut surface of the product) were observed for 60 days on the transdermal patches of each of Examples 1 to 24 and Comparative Example 1 prepared above.

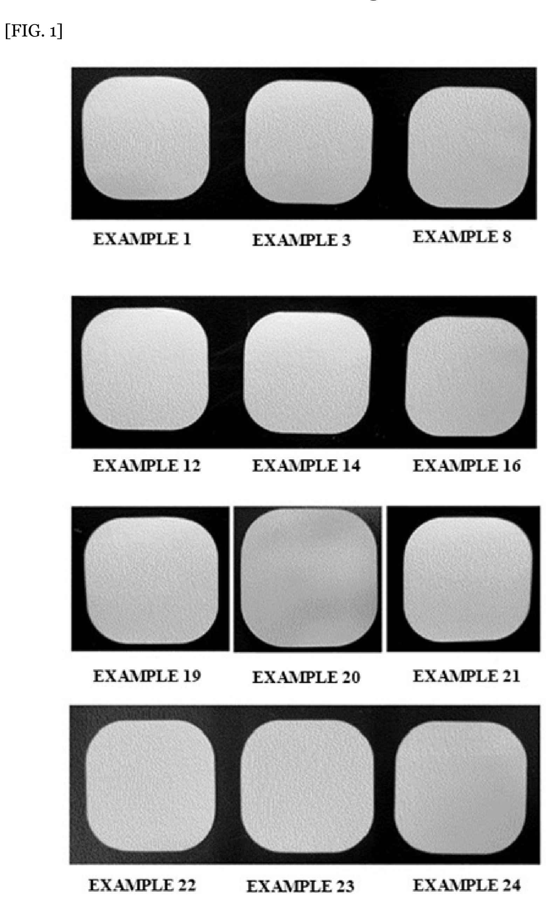

As a result, it was confirmed that no crystals precipitation occurred in any of Examples 1 to 24 containing the rotigotine and the HPC, regardless of the content of the antioxidant or the content of the silicone adhesive. Specifically, with reference to FIG. 1 illustrating the photographs of the transdermal patches according to Examples 1, 3, 8, 12, 14, 16, and 19 to 24, it can be confirmed that no crystal precipitation of the rotigotine has occurred.

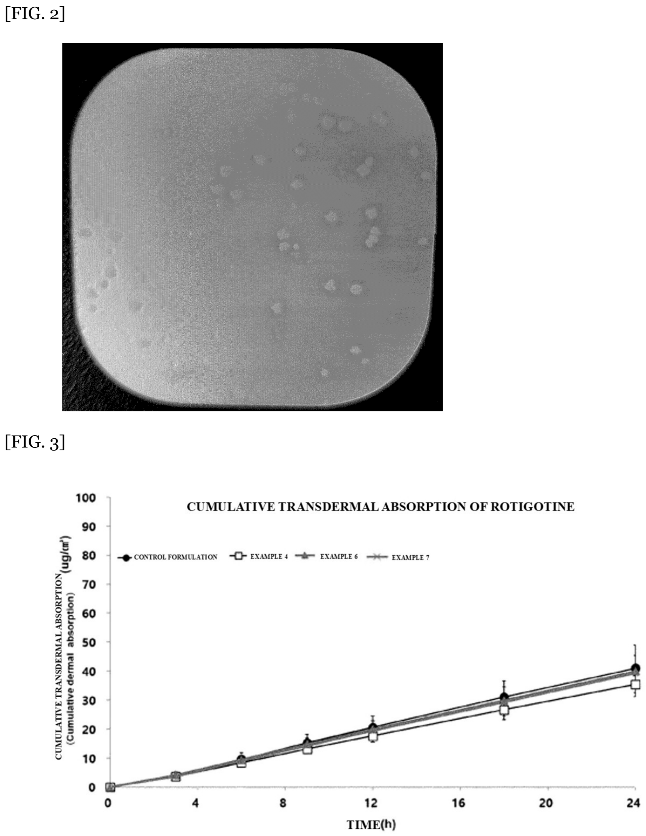

In contrast, with reference to FIG. 2 illustrating the photograph of the transdermal patch according to Comparison Example 1, it can be confirmed that the crystal precipitation of the rotigotine has occurred in Comparative Example 1 without the HPC.

Furthermore, it was also confirmed that cold flow phenomenon, where the silicone-based polymer adhesive remains on the skin adhesion surface, did not occur in the examples of the present invention.

### Experiment Example 2: In Vitro Transdermal Absorption Experiment

To evaluate the transdermal absorption performance of the transdermal patches, a transdermal absorption experiment was conducted with using Franz cell (vertical diffusion cell) with a commercially available product (NEUPRO^{®} (rotigotine transdermal system)) as the control drug, and the results are shown in FIG. 2.

With reference to FIG. 3, it can be confirmed that the transdermal patches according to Examples 4, 6, and 7 exhibited substantially equivalent transdermal absorption performance compared to the control drug.. While transdermal patches using acrylic adhesives or the like exhibit significantly reduced transdermal absorption performance, it can be confirmed that the present invention can prevent crystal precipitation, as confirmed in Experimental Example 1, while maintaining excellent transdermal absorption performance.
The present invention has been described with reference to preferred exemplary embodiments herein, but it will be understood by those skilled in the art that the present invention may be variously changed and modified without departing from the spirit and field of the present invention, as described in the following scope of patent claims. **Industrial Applicability**

The transdermal absorption formulation and the method for preparing the same according to the present invention provide the same transdermal absorption performance as that of commercialized products, even though the rotigotine is contained as an active ingredient while remaining in the formulation in a completely dissolved state, thereby minimizing the precipitation to obtain the excellent therapeutic effect using the rotigotine.

## Claims

1. A transdermal absorption formulation comprising a drug-containing adhesive layer, wherein the drug-containing adhesive layer comprises rotigotine or a pharmaceutically acceptable salt thereof as an active ingredient; hydroxypropyl cellulose (HPC) as a crystallization inhibitor; and a silicone-based polymer adhesive.

2. The transdermal absorption formulation according to claim 1, wherein a weight ratio of the rotigotine or the pharmaceutically acceptable salt thereof, as rotigotine, to the HPC is 1:0.1 to 1:10.

3. The transdermal absorption formulation according to claim 1, wherein a weight ratio of the rotigotine or the pharmaceutically acceptable salt thereof, as rotigotine, to the HPC is 9:12 to 9:15.

4. The transdermal absorption formulation according to claim 1, wherein the drug-containing adhesive layer further comprises a fatty acid as a solubilizer.

5. The transdermal absorption formulation according to claim 4, wherein a weight ratio of the rotigotine or the pharmaceutically acceptable salt thereof, as rotigotine, to the fatty acid is 9:0.01 to 9:5.

6. The transdermal absorption formulation according to claim 4, wherein a content of the hydroxypropyl cellulose is 10% to 15% by weight with respect to a total weight of the drug-containing adhesive layer, and a content of the fatty acid is 0.01% to 5% by weight with respect to the total weight of the drug-containing adhesive layer.

7. The transdermal absorption formulation according to claim 1, wherein a content of the hydroxypropyl cellulose is 10% to 15% by weight with respect to a total weight of the drug-containing adhesive layer.

8. The transdermal absorption formulation according to claim 1, wherein the drug-containing adhesive layer further comprises an antioxidant.

9. The transdermal absorption formulation according to claim 1, wherein the drug-containing adhesive layer further comprises polyvinyl pyrrolidone.

10. The transdermal absorption formulation according to claim 1, wherein the transdermal absorption formulation is a transdermal patch.

11. The transdermal absorption formulation according to claim 1, wherein the transdermal absorption formulation is a transdermal patch that comprises a backing layer and a release liner respectively disposed on both surfaces of the drug-containing adhesive layer so that the drug-containing adhesive layer is interposed between the backing layer and the release liner.

12. A method for preparing the transdermal absorption formulation according to claim 1, the method comprises:
a step of coating a backing layer with a transdermal absorption composition that comprises rotigotine or a pharmaceutically acceptable salt thereof as an active ingredient, hydroxypropyl cellulose (HPC) as a crystallization inhibitor, a silicone-based polymer adhesive, and an organic solvent; and
a step of drying a drug-containing adhesive composition coated onto the backing layer to form a drug-containing adhesive layer.

13. The method for preparing the transdermal absorption formulation according to claim 12, wherein in the step of coating the backing layer with the transdermal absorption composition, the transdermal absorption composition further comprises a fatty acid.

14. The method for preparing the transdermal absorption formulation according to claim 12, wherein the organic solvent comprises an alcohol having 1 to 5 carbon atoms.
